# EUROPÄISCHE PATENTSCHRIFT

(11) **EP 3 103 513 B1**
(45) Veröffentlichungstag und Bekanntmachung des Hinweises auf die Patenterteilung: **11.10.2023**
(21) Anmeldenummer: 16166559.1
(22) Anmeldetag: 22.04.2016
(51) Int. Cl.: A61N 1/365, A61N 1/362, A61N 1/37, A61N 1/375, A61B 5/024, A61B 5/11, A61B 5/283, A61N 1/372, A61B 5/00

(54) **RATENADAPTIVER INTRA- ODER EPICARDIALER HERZSTIMULATOR UND AKTIVITÄTSSENSOR**
RATE ADAPTIVE INTRA- OR EPICARDIAL HEART STIMULATOR AND ACTIVITY SENSOR
STIMULATEUR CARDIAQUE EPICARDIQUE OU INTRACARDIAQUE SENSIBLE AU RYTHME ET CAPTEUR D'ACTIVITES

(30) Priorität: 09.06.2015 DE 102015109037
(43) Veröffentlichungstag der Anmeldung: 14.12.2016
(73) Patentinhaber: BIOTRONIK SE & Co. KG, 12359 Berlin (DE)
(72) Erfinder: Busch, Ulrich, 10318 Berlin (DE); Neumann, Andreas, 12437 Berlin (DE)
(74) Vertreter: Biotronik Corporate Services SE

(56) Entgegenhaltungen:
- EP-A1- 2 065 071
- WO-A1-01/56651
- WO-A1-2015/100071
- US-A- 5 179 947
- US-A1- 2012 095 521
- US-A1- 2012 109 236
- US-A1- 2013 079 861

## Beschreibung

Die Erfindung betrifft einen elektrodenleitungslosen, intra- oder epicardialen Herzstimulator mit einem Aktivitätssensor. Außerdem betrifft die Erfindung einen Aktivitätssensor zum Erfassen einer körperlichen Aktivität anhand erfasster Beschleunigungswerte.

Implantierbare Herzstimulatoren in ihrer grundsätzlich bekannten Form als Herzschrittmacher besitzen ein Gehäuse, in welchem eine Batterie angeordnet ist, die einen Betrieb des Schrittmachers über viele Jahre erlaubt. Die Batterie versorgt eine Schrittmachersteuerung, die in der Regel mit einer Erfassungsstufe für Herzsignale sowie mit einem Stimulationsimpulsgenerator verbunden ist. Sowohl der Stimulationsimpulsgenerator als auch die Erfassungsstufe für Herzsignale sind ihrerseits wiederum mit Anschlüssen für eine oder mehrere Elektrodenleitungen verbunden, die es erlauben, vom Stimulationsimpulsgenerator erzeugte Stimulationsimpulse an das Muskelgewebe des Herzens (Myokard) abzugeben oder elektrische Potentiale im Herz zu erfassen und zu verarbeiten.

Neben solchen Herzschrittmachern, die typischerweise unterhalb des Schlüsselbeins eines Patienten implantiert werden und bei denen eine Verbindung zum Herzen über flexible Elektrodenleitungen erfolgt, die in der jeweiligen Herzkammer (Atrium oder Ventrikel) enden und dort mit Elektroden versehen sind, sind auch sogenannte leadless Herzschrittmacher bekannt, die als ganzes in einer Herzkammer (intracardial) platziert werden können. Auch außerhalb am Herzen (epicardial) zu befestigende Herzschrittmacher sind angedacht.

Da am oder im Herzen zu platzierende Herzstimulatoren keine Elektrodenleitungen benötigen, werden sie als "leadless" bezeichnet. Im Rahmen dieser Beschreibung werden solche Herzschrittmacher daher als leadless Herzschrittmacher oder synonym als elektrodenleitungsloser Herzstimulator bezeichnet.

Die verschiedenen Betriebsweisen und Varianten von Herzschrittmachern sind grundsätzlich bekannt und brauchen hier nicht weiter erläutert zu werden. Insbesondere ist es bekannt, eine Stimulationsrate eines Herzschrittmachers in Abhängigkeit des metabolischen Bedarfs eines Patienten zu steuern. Derartige ratenadaptive Herzschrittmacher benötigen einen Sensor für den metabolischen Bedarf. Mögliche Sensortypen sind Temperatursensoren oder Beschleunigungssensoren (Akzelerometer).

Ein ratenadaptiver Herzschrittmacher mit einem Beschleunigungssensor ist z.B. in US 4,926,863 beschrieben. US 8,543,205 B2 beschreibt einen ratenadaptiven intracardialen Herzschrittmacher mit einem Temperatursensor. US 7,937,148 B2 beschreibt einen ratenadaptiven intracardialen Herzschrittmacher mit einem Beschleunigungssensor.

Die Patentanmeldungen US 2012/109236 A1, WO 2015/100071 A1 und WO 2001/56651 A1 beschreiben ebenfalls ratenadaptive intrakardiale Herzschrittmacher mit einem Beschleunigungssensor.

Die Druckschrift EP 2 065 071 A1 betrifft eine intrakardial implantierare Elektrodenleitung zum Anschluss an ein implantierbares medizinisches Gerät, insbesondere einen Herzschrittmacher oder Kardioverter/Defibrillator oder dergleichen, die im Bereich ihres distalen Endes einen Beschleunigungssensor aufweist, der zur Aufnahme und Unterscheidung von Beschleunigungswerten in wenigstens zwei unterschiedlichen Richtungen ausgebildet ist. Außerdem betrifft die Erfindung eine Herzstimulationsanordnung, die neben einer solchen Elektrodenleitung auch einen Herzstimulator aufweist.

Der Erfindung liegt die Aufgabe zugrunde, einen intra- oder epicardial zu platzierenden Herzschrittmacher mit Beschleunigungssensor zu schaffen, der es ermöglicht, die Körperaktivität des Patienten zu bestimmen.

Erfindungsgemäß wird zur Lösung dieser Aufgabe ein elektrodenleitungsloser implantierbarer Herzstimulator mit einem Beschleunigungssensor vorgeschlagen, der als Ausgangssignal ein Beschleunigungssignal liefert. Das Beschleunigungssignal ist dabei ein Summensignal eines auf eine Herzeigenbewegung zurückgehenden Anteils und eines auf eine Körperbewegung zurückgehenden Anteils. Der Beschleunigungssensor ist mit einer Aktivitätsbestimmungseinheit verbunden, die ausgebildet ist, aus dem Beschleunigungssignal ein Aktivitätssignal zu generieren, indem die Aktivitätsbestimmungseinheit den auf eine Herzeigenbewegung zurückgehenden Anteil des Beschleunigungssignals von dem Beschleunigungssignal subtrahiert. Der erfindungsgemäße elektrodenleitungslose implantierbare Herzstimulator zeichnet sich dadurch aus, dass das Beschleunigungssignal vorzeichenbehaftet ist und die die Aktivitätsbestimmungseinheit zur Generation des Aktivitätssignals dazu ausgebildet ist, das Beschleunigungssignal über einen vorgegebenen Zeitraum zu integrieren um so ein Beschleunigungsintegral zu bilden.

Die Erfindung schließt die Erkenntnis ein, dass ein im oder am Herzen platzierter Beschleunigungssensor sowohl die Herzeigenbewegung als auch die Bewegung des gesamten Körpers registriert, weil ein leadless Herzschrittmacher nicht nur Körperbewegungen, sondern auch Herzeigenbewegungen folgt. Das Beschleunigungssignal enthält somit Signalanteile die auf Körperbewegungen zurückgehen und von Herzeigenbewegungen überlagert sind, die eine Störfunktion darstellen. Das Beschleunigungssignal ist somit Summensignal der beiden Bewegungskomponenten, Herzeigenbewegung und Körperbewegung. Zur Steuerung der Stimulationsrate ist eine Trennung der beiden Signalkomponenten notwendig. Die Trennung der Signalkomponenten kann mit unterschiedlichen Methoden erfolgen, die gemeinsam haben, dass ein Mittelwert der Beschleunigung der Herzkomponente bestimmt wird.

Vorteil eines erfindungsgemäßen Herzstimulators ist, dass eine Stimulationsratenanpassung auf Basis eines Beschleunigungssignals erfolgen kann, das von einem Beschleunigungssensor stammt. Beschleunigungssensoren sind als Sensoren für die Stimulationsratenanpassung eines Herzstimulators bekannt. Beschleunigungssensoren sprechen schnell an, haben kaum Stabilitätsprobleme und ihr Verhalten ist leicht nachzuvollziehen. Mittels einer erfindungsgemäßen Aktivitätsbestimmungseinheit können Beschleunigungssensoren auch für die Stimulationsratenanpassung in einem elektrodenleitungslosen Herzstimulator verwendet werden.

Die Aktivitätsbestimmungseinheit ist vorzugsweise ausgebildet, das Beschleunigungsintegral jeweils über einen Herzzyklus zu bilden.

Der Herzstimulator weist vorzugsweise eine Steuereinheit auf, die mit der Aktivitätsbestimmungseinheit verbunden und ausgebildet ist, Ruhephasen zu erfassen. Außerdem weist der Herzstimulator vorzugsweise wenigstens eine Sensingeinheit auf, die mit wenigstens einer Elektrode verbunden und ausgebildet ist, über die Elektrode elektrische Potentialverläufe als iEKG-Signal aufzunehmen und für Herzeigenaktionen charakteristische Signalmerkmale, insbesondere R-Zacken, des iEKG-Signals zu detektieren.

Vorzugsweise bildet der Herzstimulator einen ratenadaptiven Herzschrittmacher, indem der Herzstimulator eine Steuereinheit und eine mit dieser verbundene Stimulationseinheit aufweist und ausgebildet ist, die Stimulationseinheit auf Basis einer Stimulationsrate anzusteuern, die von dem Aktivitätssignal abhängig ist.

Der Herzstimulator weist beispielsweise ein intracardial oder epicardial zu platzierendes Gehäuse auf.

Das Gehäuse trägt beispielsweise wenigstens zwei Elektroden, die jeweils eine nach außen gerichtete, elektrisch leitende Oberfläche aufweisen und als Stimulationselektroden ausgebildet sowie wenigstens zeitweise mit dem Stimulationsimpulsgenerator elektrisch über eine im Inneren des Gehäuses angeordnete elektrische Verbindung und ein elektrisches Schaltelement verbunden sind. Als Elektroden werden in diesem Zusammenhang jene elektrisch leitenden Bauelemente bezeichnet, über die beispielsweise Stimulationsimpulse abgegeben oder auch elektrische Potentiale erfasst werden können. Die Elektroden bilden somit mit ihrer elektrisch leitenden Oberfläche Pole für die Stimulation. Sie sind bei dem Herzschrittmacher in dessen Gehäuse integriert.

Ein derartiger Herzschrittmacher hat die Eigenschaft, beispielsweise in dem vom rechten Atrium aus zugänglichen Herzohr oder direkt im Ventrikel angeordnet werden zu können und aufgrund der auf seiner Oberfläche angeordneten Stimulationselektroden als autarkes Gerät wenigstens das Atrium oder den Ventrikel stimulieren zu können.

Von den Elektroden (Polen) ist beispielsweise wenigstens eine erste Elektrode sehr kleinflächig - also beispielsweise punktförmig - und besitzt eine Oberfläche von weniger als 5 mm². Diese erste Elektrode ist beispielsweise an einem Längsende des längsgestreckten Gehäuses angeordnet. Auch die zweite Elektrode kann kleinflächig sein und beispielsweise die gleiche Form besitzen, wie die erste Elektrode. Beispielsweise sind beide Elektroden in unmittelbarer Nachbarschaft zueinander am selben Längsende des Gehäuses angeordnet.

Die zweite Elektrode kann aber auch von einem großflächigen Gehäuseteil des Gehäuses des Herzschrittmachers gebildet sein und bildet so einen indifferenten Pol, der quasi als Neutralelektrode wirkt.

Beispielsweise sind die Elektroden als Ringelektroden ausgebildet, die um den Umfang des Gehäuses umlaufen. Auch in diesem Fall sind die Elektroden vorzugsweise einander dicht benachbart angeordnet. Auf diese Weise ist die genaue Ausrichtung des Herzschrittmachers nach Implantation beispielsweise im rechten Herzohr oder auch im Ventrikel relativ unkritisch.

Der Herzstimulator ist beispielsweise als Einkammer-Demand-Schrittmacher ausgebildet (AAI oder VVI). Zu diesem Zweck dienen die Elektroden vorteilhafterweise zusätzlich als Abfühlelektroden, die wenigstens zeitweise mit einer Erfassungsstufe im Inneren des Gehäuses zu verbinden sind.

Um die Funktionalität des an sich autarken Herzstimulators zu erweitern, ist es möglich, dass der Herzstimulator einen Telemetriesender und einen Telemetrieempfänger besitzt, der mit der Schrittmachersteuerung verbunden ist und es erlaubt, Betriebsdaten des Herzstimulators sowie erfasste, physiologische Daten, beispielsweise intrakardiale Elektrokardiogramme, an ein externes Gerät zu übertragen und umgekehrt Programmier- und Steuerbefehle zu empfangen. Auch Betriebsparameter, beispielsweise wie eine jeweils zu wählende Stimulationsimpulsstärke können auf diese Weise mittels eines externen Gerätes ermittelt und an den Herzstimulator übertragen werden, so dass auch die Betriebsparameter dann für den weiteren, auch autarken Schrittmacherbetrieb in einem entsprechenden Speicher der Schrittmachersteuerung zu speichern sind.

Um mit einem im Herzen selbst implantierbaren Herzstimulator nicht nur das rechte Atrium, sondern beispielsweise auch den rechten oder linken Ventrikel stimulieren zu können, besitzt der Herzstimulator vorzugsweise eine drahtgebundene oder besser noch drahtlose Schnittstelle, die mit der Schrittmachersteuerung verbunden und ausgebildet ist, mit einer entsprechenden Schnittstelle eines zweiten Schrittmachers Daten bezüglich der Zeitpunkte in einer jeweiligen Herzkammer auftretender Ereignisse (natürliche oder stimulierte Kammerkontraktionen) auszutauschen. Auf diese Weise wird ein System aus zwei oder mehr Herzstimulatoren möglich, von denen einer im Atrium und einer im Ventrikel platziert werden kann und die zusammen die Funktionalität eines Zweikammerschrittmachers besitzen. In einem solchen System übermittelt der atriale Herzstimulator dem ventrikulären Herzstimulator die Zeitpunkte atrialer Ereignisse und vice versa. Somit kann die Steuerung des jeweils einen Herzstimulators auf Ereignisse in der jeweils anderen Herzkammer ansprechen, also beispielsweise der ventrikuläre Herzstimulator auf atriale Ereignisse, so dass eine an sich bekannte, atriumsynchrone Stimulation des Ventrikels mit physiologisch adäquater atrioventrikulärer Verzögerungszeit (AV-Intervall) möglich ist.

Mit mehr als zwei intrakardial implantierbaren Herzstimulatoren der erfindungsgemäßen Art ist beispielsweise auch eine Dreikammerstimulation im Rahmen einer Resynchronisations-Therapie möglich.

Anstelle einer Zweikammerfunktion wie zuvor beschrieben mit zwei miteinander datenverbundenen Herzstimulatoren zu verwirklichen, kann einem in einer Herzkammer zu platzierenden Herzstimulator auch eine Elektrodenleitung für eine jeweils andere Herzkammer angeschlossen sein. Diese Elektrodenleitung ist biegeweich und trägt wenigstens eine Stimulationselektrode an ihrem freien Ende oder in der Nähe ihres freien Endes. Ihre Länge ist so bemessen, dass sie entweder über den Coronar Sinus und gegebenenfalls eine davon abzweigende Lateralvene eine Stimulation des linken Ventrikels erlaubt oder durch das rechte Atrium hindurch in den rechten Ventrikel ragt. Bevorzugt ist eine Elektrodenleitung, die in den Coronar Sinus und in eine vom Coronar Sinus abzweigende Lateralvene einzuführen ist. Die Elektrodenleitung ist vorzugsweise dauerhaft an das Gehäuse des Herzschrittmachers angeschlossen und besitzt in jedem Fall einen elektrischen Leiter, der die Stimulationselektrode der Elektrodenleitung mit dem Stimulationsimpulsgenerator des Herzstimulators verbindet. Auf diese Weise wird der Herzstimulator zum Zweikammer-Schrittmacher, der für die Stimulation der Herzkammer, in der er im Anwendungsfall platziert ist, Elektroden auf seinem Gehäuse trägt und der zur Stimulation der anderen Herzkammer die biegeweiche Elektrodenleitung besitzt. Um eine Zweikammer-Stimulation zu ermöglichen, kann der Herzstimulator einen einzigen Stimulationsimpulsgenerator besitzen, der abwechselnd mit den atrialen Stimulationselektroden und den ventrikulären Stimulationselektroden zu verbinden ist. Es können auch zwei separate Stimulationsimpulsgeneratoren für das Atrium und den Ventrikel vorgesehen sein, die mit einer Leitung verbunden sind.

Bei einem Herzstimulator, der einen beispielsweise kreisrunden Querschnitt besitzt und dessen Gehäuse beispielsweise wenigstens über einen Teil seiner Länge zylindrisch geformt ist, kann in vorteilhafter- und das Gehäusevolumen bestmöglich ausnutzender Weise eine Batterie Verwendung finden, deren Querschnitt ebenfalls beispielsweise rund ist. Dies erlaubt es in besonders vorteilhafter Weise, die Elektroden der Batterie in an sich bekannter Art und Weise zu wickeln. Das anzustrebende Batterievolumen beträgt zwischen 1 cm³ und 3 cm³. Die Batteriekapazität liegt vorzugsweise zwischen 0,25 und 0,75 Ah.

Das Gehäuse wird beispielsweise von miteinander verschweißten Gehäuseelementen aus Metall gebildet. Wenigstens eine der Elektroden ist gegenüber dem verschweißten MetallGehäuse elektrisch isoliert. Von den Metall-Gehäuseelementen ist beispielsweise eines röhrenförmig mit geschlossenem Boden ausgeführt. In dieses Gehäuseelement können Batterie und Steuerelektronik eingesetzt werden. Dies erlaubt es das Gehäuse aus beispielsweise drei Gehäuseelementen zusammenzusetzen, nämlich aus einem zentralen, röhrenförmigen Gehäuseelement sowie zwei Endkappen. Die Stimulations- und/oder Abfühlelektroden können dann jeweils zwischen einer der Gehäusekappen und dem zentralen, röhrenförmigen Gehäuseelement angeordnet werden und sind an diesem Ort auch leicht zu montieren.

Alternativ können die Gehäuseelemente aus Keramik oder Kunststoff gefertigt sein und die Elektroden können von Metallringen gebildet sein.

Die Erfindung soll nun anhand eines Ausführungsbeispiels näher erläutert werden. Der Illustration des Ausführungsbeispiels dienen Figuren, von denen
- Fig. 1:: eine Außenansicht einer ersten Variante eines im rechten Herzohr oder im Ventrikel zu implantierenden Herzstimulators als Einkammer-Herzschrittmacher ist;
- Fig. 2:: eine perspektivische Explosionszeichnung des Herzstimulators aus Figur 1;
- Fig. 3:: ein Blockschaltbild des Herzstimulators aus Figuren 1 und 2; und
- Fig. 4:: ein von zwei implantierten Herzschrittmachern gebildetes Zweikammer-Schrittmachersystem.

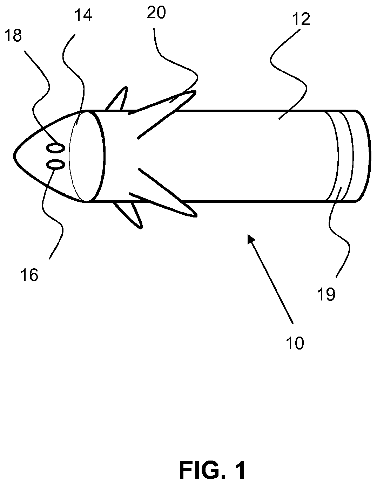

In Figur 1 ist ein Herzstimulator 10 in Form eines Einkammer-Demand-Herzschrittmachers abgebildet. Der Herzstimulator 10 besitzt ein längs gestrecktes, zylindrisches Gehäuse 12 mit einer halbkugelförmigen Gehäusekappe 14. An der Spitze der Gehäusekappe 14 sind zwei kleinflächige Stimulationselektroden 16 und 18 angeordnet. Um den Herzstimulator 10 in einer Herzkammer wie beispielsweise dem Ventrikel 56 oder auch im Herzohr des rechten Atriums zu fixieren, sind an dem Gehäuse 12 Arretierungshaken 20 vorgesehen.

Das Gehäuse 12 ist dicht mit der Gehäusekappe 14 verbunden, so dass der von dem Gehäuse 12 und der Gehäusekappe 14 eingeschlossene Raum hermetisch abgeschlossen ist. Die Elektroden 16 und 18 sind in die Gehäusekappe 14 eingelassen und besitzen elektrisch leitende Oberflächen, die gegenüber dem übrigen Gehäuse 12 elektrisch isoliert sind und als Pole für die Stimulation von Herzgewebe dienen. Außerdem ist eine Fernfeld-Sensingelektrode 19 als Ringelektrode vorgesehen.

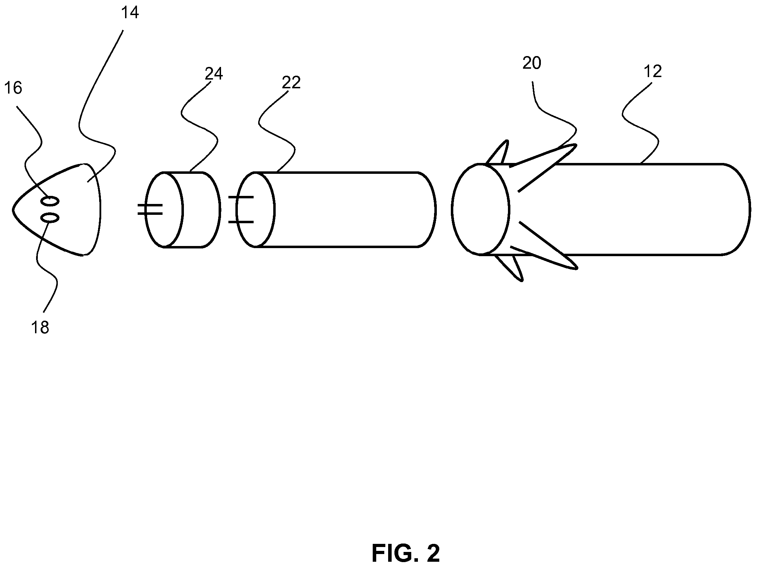

Figur 2 ist eine Explosionszeichnung des Herzstimulators 10 aus Figur 1 und zeigt die im Inneren des Gehäuses 12 angeordneten Bauteile, nämlich eine Batterie 22 sowie ein Elektronikmodul 24. Das Elektronikmodul 24 umfasst eine elektronische Schrittmachersteuerung sowie wenigstens einen Stimulationsimpulsgenerator und im Falle der Ausbildung des Herzstimulators als Demand-Schrittmacher außerdem eine Erfassungsstufe für Herzsignale als Sensingeinheit. Der Stimulationsimpulsgenerator sowie die Sensingeinheit sind wechselweise jeweils mit den beiden Elektroden 16 und 18 elektrisch verbunden.

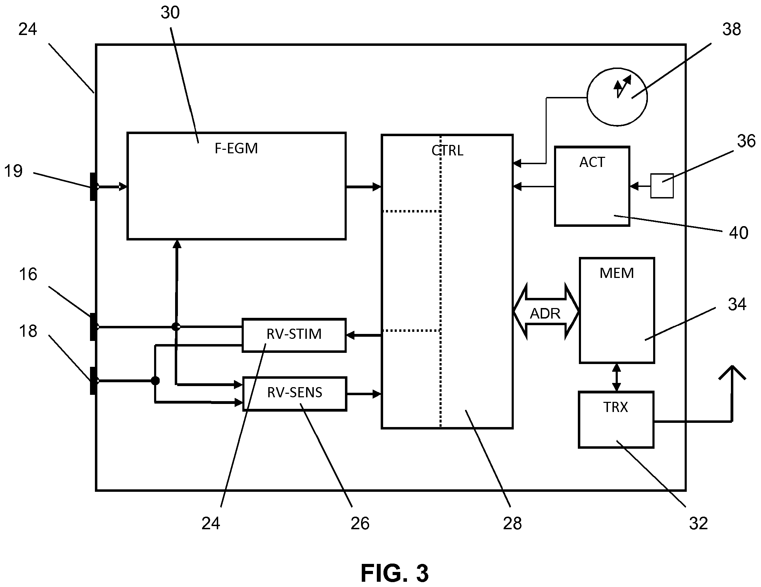

In Fig. 3 sind einige Komponenten des Elektronikmoduls 24 schematisch dargestellt.

Die Elektroden 16 und 18 sind jeweils sowohl mit einem Stimulationsimpulsgenerator 25 als auch mit einer Sensingeinheit 26 verbunden. Bei Verwendung des Herzstimulators 10 als rechtsventrikulären Herzschrittmacher ist der Stimulationsimpulsgenerator 25 eine rechtsventrikuläre Stimulationseinheit und die Sensingeinheit 26 eine rechtsventrikuläre Sensingeinheit. Der Stimulationsimpulsgenerator 25 ist ausgebildet, die Energie für einen Stimulationsimpuls bereitzustellen und auf ein Auslösesignal hin über die Stimulationselektrode 16 an anliegendes Gewebe abzugeben. Hierzu ist der Stimulationsimpulsgenerator 25 mit einer Steuereinheit 28 verbunden, die ein entsprechendes Auslösesignal generiert.

Die Sensingeinheit 26 kann über die Elektroden 16 und 18 lokale Herzereignisse erfassen, das heißt Herzereignisse, die der Kammer zuzuordnen sind, die benachbart zu dem epikardialen Herzstimulator 10 angeordnet ist. Im Falle eines ventrikulären Herzschrittmachers erfasst die Sensingeinheit 26 somit rechtsventrikuläre Ereignisse und gibt beim Erfassen eines jeweiligen ventrikulären Ereignisses ein entsprechendes Ausgangssignal an die Steuereinheit 28 aus. Dies erlaubt es der Steuereinheit 28, Stimulationsimpulse nur im Bedarfsfall auszulösen, nämlich dann, wenn die Sensingeinheit 26 kein intrinsisches ventrikuläres Ereignis erfasst hat.

Die Fernfeld-Sensingelektrode 19 ist mit einer Fernfeld-Sensingeinheit 30 verbunden, die über einen weiteren Eingang mit der Stimulationselektrode 16 verbunden ist. Die Fernfeld-Sensingeinheit 30 ist ausgebildet, die Potentialdifferenz zwischen der Fernfeld-Sensingelektrode 19 und der Stimulationselektrode 16 im Sinne eines Fernfeld-Elektrokardiogramms auszuwerten. Die Fernfeld-Sensingeinheit 30 liefert an die Steuereinheit 28 ein Ausgangssignal, welches Signalmerkmale enthält, die beispielsweise eine atriale Aktivität signalisieren. Mit Hilfe der Fernfeld-Sensingeinheit 30 ist es der Steuereinheit 28 somit möglich, auch beispielsweise atriale Ereignisse zu erfassen, wenn der Herzstimulator 10 als ventrikulärer Herzschrittmacher verwendet und entsprechend, beispielsweise dem rechten Ventrikel 56 benachbart, angeordnet ist.

Mit dem Herzstimulator 10 ist somit beispielsweise eine atrium synchrone bedarfsabhängige ratenadaptive Stimulation des rechten Ventrikels 56 möglich.

In Fig. 3 ist außerdem angedeutet, dass die Steuereinheit mit einer Speichereinheit 34 verbunden sein kann, die beispielsweise dem Speichern von Steuersignalen dient oder aber auch dem Speichern von Signalverläufen, die der Herzstimulator 10 aufgenommen hat. Um den Herzstimulator 10 umprogrammieren zu können oder aufgenommene Signalverläufe zur Auswertung an ein externes Gerät übertragen zu können, weist der Herzstimulator 10 außerdem eine Telemetrieeinheit 32 auf, die mit dem Speicher 34 verbunden ist. Um eine dem metabolischen Bedarf eines jeweiligen Patienten angemessene Stimulationsrate einstellen zu können, ist die Steuereinheit 28 außerdem mit einem Aktivitätssensor 36 in Form eines Beschleunigungssensors verbunden, so dass sich ein ratenadaptiver Herzschrittmacher ergibt. Der Aktivitätssensor 36 ist ein Beschleunigungssensor, der ein Beschleunigungssignal als Ausgangssignal liefert, welches vom metabolischen Bedarf eines Patienten abhängt. Typischerweise ist die Steuereinheit 28 auch mit einer Zeitgebereinheit 38 verbunden.

Der Herzstimulator 10 weist außerdem eine Aktivitätsbestimmungseinheit 40 auf, die das von dem Aktivitätssensor 36 gelieferte Beschleunigungssignal verarbeitet und aus dem Beschleunigungssignal ein Aktivitätssignal generiert.

Hierzu führt die Aktivitätsbestimmungseinheit 40 beispielsweise das folgende Verfahren aus:
Die Aktivitätsbestimmungseinheit 40 verarbeitet das vorzeichenbehaftete Beschleunigungssignal. Zur Trennung der beiden Signalkomponenten wird eine Mittelwertbildung genutzt, die das Beschleunigungssignal über jeden einzelnen kompletten Herzzyklus aufaddiert. Die positiven und negativen Anteile des Beschleunigungssignals eines Herzzyklus heben sich weitgehend auf. Dadurch wird die Komponente der Herzeigenbewegung eliminiert. Das übrigbleibende Beschleunigungssignal wird wie aus dem Stand der Technik bekannt zur Stimulationsratenanpassung verwendet. Bei dieser Verfahrensvariante wird die nutzbare Bandbreite der Körperkomponente des Beschleunigungssignals durch die Herzfrequenz nach oben hin begrenzt. Der Herzzyklus kann jeweils leicht mit Hilfe der R-Zacken bestimmt werden, die ihrerseits wiederum mittels der Sensingeinheit 26 erfasst werden.

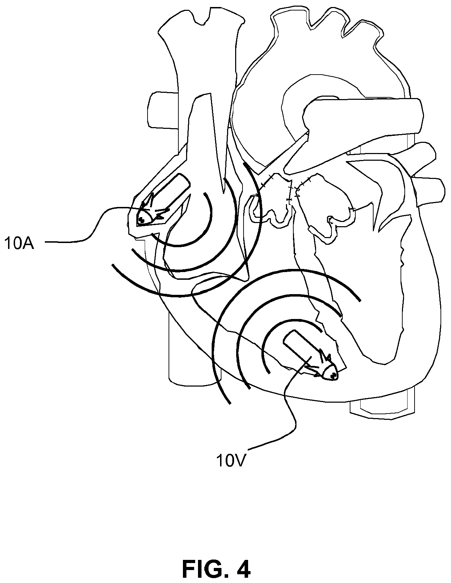

Wie zuvor bereits erwähnt, kann das Elektronikmodul 24 eines jeweiligen Herzstimulators eine drahtgebundene oder besser noch drahtlose Schnittstelle zu einem oder mehr weiteren Herzstimulator der gleichen Art besitzen, so dass zwei oder mehr Herzstimulatoren gemeinsam ein Mehrkammer-Schrittmachersystem bilden können, wie es in Figur 4 abgebildet ist. In Figur 4 ist ein Zweikammersystem abgebildet. Ein erster Herzstimulator 10A ist im rechten Herzohr des rechten Atriums 52 angeordnet, während ein zweiter Herzstimulator 10V im rechten Ventrikel 56 des Herzens 50 angeordnet ist. Genauso kann auch nur ein einzelner Herzstimulator in einer Kammer eines Herzens vorgesehen sein und z.B. als ventrikulärer Schrittmacher im VVI Modus oder als atrialer Schrittmacher im AAI Modus operieren.

### Bezugszeichenliste

- 10: Herzstimulator
- 10A: erster Herzstimulator
- 10V: zweiter Herzstimulator
- 12: Gehäuse
- 14: Gehäusekappe
- 16,18: Stimulations- und Sensingelektroden
- 19: Fernfeld-Sensingelektrode
- 20: Arretierungshaken
- 22: Batterie
- 24: Elektronikmodul
- 25: Stimulationsimpulsgenerator
- 26: Sensingeinheit
- 28: Steuereinheit
- 30: Fernfeld-Sensingeinheit
- 32: Telemetrieeinheit
- 34: Speicher
- 36: Beschleunigungssensor
- 38: Zeitgebereinheit
- 40: Aktivitätsbestimmungseinheit
- 50: Herz
- 52: rechtes Atrium
- 56: Ventrikel

## Patentansprüche

1. Elektrodenleitungsloser implantierbarer Herzstimulator (10) mit einem Beschleunigungssensor (36), der als Ausgangssignal ein Beschleunigungssignal liefert, wobei das Beschleunigungssignal ein Summensignal eines auf eine Herzeigenbewegung zurückgehenden Anteils und eines auf eine Körperbewegung zurückgehenden Anteils ist und der Beschleunigungssensor (36) mit einer Aktivitätsbestimmungseinheit (40) verbunden ist, die ausgebildet ist, aus dem Beschleunigungssignal ein Aktivitätssignal zu generieren, indem die Aktivitätsbestimmungseinheit (40) den auf die Herzeigenbewegung zurückgehenden Anteil des Beschleunigungssignals von dem Beschleunigungssignal subtrahiert,
**dadurch gekennzeichnet, dass**
das Beschleunigungssignal vorzeichenbehaftet ist und die Aktivitätsbestimmungseinheit (40) zur Generation des Aktivitätssignals dazu ausgebildet ist, das Beschleunigungssignal über einen vorgegebenen Zeitraum zu integrieren um so ein Beschleunigungsintegral zu bilden.

2. Herzstimulator (10) gemäß Anspruch 1, **dadurch gekennzeichnet, dass** die Aktivitätsbestimmungseinheit (40) ausgebildet ist, das Beschleunigungsintegral jeweils über einen Herzzyklus zu bilden.

3. Herzstimulator (10) gemäß einem der Ansprüche 1 oder 2, **dadurch gekennzeichnet, dass** der Herzstimulator (10) eine Steuereinheit aufweist, die mit der Aktivitätsbestimmungseinheit (40) verbunden und ausgebildet ist, Ruhephasen zu erfassen.

4. Herzstimulator (10) gemäß einem der Ansprüche 1 bis 3, **dadurch gekennzeichnet, dass** der Herzstimulator (10) wenigstens eine Sensingeinheit (26) aufweist, die mit wenigstens einer Elektrode verbunden und ausgebildet ist, über die Elektrode elektrische Potentialverläufe als iEKG-Signal aufzunehmen und für Herzeigenaktionen charakteristische Signalmerkmale, insbesondere R-Zacken, des iEKG-Signals zu detektieren.

5. Herzstimulator (10) gemäß einem der Ansprüche 1 bis 4, **dadurch gekennzeichnet, dass** der Herzstimulator (10) eine Steuereinheit und eine mit dieser verbundene Stimulationseinheit aufweist und ausgebildet ist, die Stimulationseinheit auf Basis einer Stimulationsrate anzusteuern, die von dem Aktivitätssignal abhängig ist.

## Claims

1. An electrode leadless implantable heart stimulator (10) comprising an acceleration sensor (36) that delivers an acceleration signal as an output signal, wherein the acceleration sensor is a sum signal of a component caused by an intrinsic heart movement and of a component caused by a body movement and the acceleration signal (36) is connected to an activity determination unit (40) which is configured to generate an activity signal from the acceleration signal as a result of the activity determination unit (40) subtracting the component of the acceleration signal caused by the intrinsic heart movement from the acceleration signal,
**characterised in that**
the acceleration signal is a signed signal and the activity determination unit (40) for generation of the activity signal is configured to integrate the acceleration signal over a predefined period of time in order to thus form an acceleration integral.

2. The heart stimulator (10) according to claim 1, **characterised in that** the activity determination unit (40) is configured to form the acceleration integral in each case over a cardiac cycle.

3. The heart stimulator (10) according to any one of claims 1 or 2, **characterised in that** the heart stimulator (10) has a control unit which is connected to the activity determination unit (40) and is configured to detect rest phases.

4. The heart stimulator (10) according to any one of claims 1 to 3, **characterised in that** the heart stimulator (10) has at least one sensing unit (26), which is connected to at least one electrode and is configured to record electrical potential curves via the electrode as an iECG signal and to detect signal features, in particular R-peaks, of the iECG signal that are characteristic of intrinsic heart actions.

5. The heart stimulator (10) according to any one of claims 1 to 4, **characterised in that** the heart stimulator (10) has a control unit and a stimulation unit connected thereto and is configured to control the stimulation unit on the basis of a stimulation rate which is dependent on the activity signal.

## Revendications

1. Stimulateur cardiaque (10) implantable sans ligne d'électrode pourvu d'un capteur d'accélération (36) qui délivre un signal d'accélération en tant que signal de sortie, dans lequel le signal d'accélération est un signal somme d'une contribution issue d'un mouvement propre au coeur et d'une contribution issue d'un mouvement du corps, et le capteur d'accélération (36) est relié avec une unité de détermination d'activité (40) qui est conçue pour générer un signal d'activité à partir du signal d'accélération en ce que l'unité de détermination d'activité (40) soustraie la contribution issue du mouvement propre au coeur du signal d'accélération,
**caractérisé en ce que**
le signal d'accélération est muni d'un signe et l'unité de détermination d'activité (40) pour la génération du signal d'activité est conçue pour intégrer le signal d'accélération sur un intervalle de temps prédéfini afin de former ainsi une intégrale d'accélération.

2. Stimulateur cardiaque (10) selon la revendication 1, **caractérisé en ce que** l'unité de détermination d'activité (40) est conçue pour former l'intégrale d'accélération respectivement sur un cycle cardiaque.

3. Stimulateur cardiaque (10) selon l'une des revendications 1 ou 2, **caractérisé en ce que** le stimulateur cardiaque (10) présente une unité de commande qui est reliée avec l'unité de détermination d'activité (40) et est conçue pour détecter des phases de repos.

4. Stimulateur cardiaque (10) selon l'une des revendications 1 à 3, **caractérisé en ce que** le stimulateur cardiaque (10) présente au moins une unité de détection (26) qui est reliée avec au moins une électrode et est conçue pour enregistrer des évolutions de potentiels électriques sous forme de signaux d'EGC par le biais de l'électrode et pour détecter des particularités de signaux caractéristiques d'actions propres au coeur, notamment des pics de R du signal d'ECG.

5. Stimulateur cardiaque (10) selon l'une des revendications 1 à 4, **caractérisé en ce que** le stimulateur cardiaque (10) présente une unité de commande et une unité de stimulation reliée à celle-ci et est conçu pour démarrer l'unité de stimulation sur la base d'un taux de stimulation qui dépend du signal d'activité.
